# EUROPEAN PATENT SPECIFICATION

(11) **EP 2 078 025 B1**
(45) Date of publication and mention of the grant of the patent: **13.04.2016**
(21) Application number: 07820533.3
(22) Date of filing: 24.09.2007
(51) Int. Cl.: C07D 495/04, A61K 31/4365, A61P 7/02

(54) **PROCESS FOR THE SYNTHESIS OF CLOPIDOGREL AND NEW FORMS OF PHARMACEUTICALLY ACCEPTABLE SALTS THEREOF**
VERFAHREN ZUR SYNTHESE VON CLOPIDOGREL UND NEUE FORMEN VON PHARMAZEUTISCH UNBEDENKLICHEN SALZEN DAVON
PROCÉDÉ DE SYNTHÈSE DU CLOPIDOGREL ET NOUVELLES FORMES DE SELS PHARMACEUTIQUEMENT ACCEPTABLES DE CELUI-CI

(30) Priority: 22.09.2006 SI 200600220
(43) Date of publication of application: 15.07.2009
(73) Proprietor: KRKA, tovarna zdravil, d.d., Novo mesto, 8501 Novo mesto (SI)
(72) Inventor: SIMONIC, Igor, 8351 Straza (SI); BENKIC, Primoz, SLO-1000, Ljubljana (SI); ZUPET, Rok, 1211 Ljubljana (SI); SMRKOLJ, Matej, 1420 Trbovje (SI); STUKELJ, Mitja, 8000 Novo Mesto (SI)
(74) Representative: Uexküll & Stolberg
(86) International application number: PCT/EP2007/060127
(87) International publication number: WO 2008/034912

(56) References cited:
- WO-A-02/059128
- WO-A-03/051362
- WO-A-2004/013147
- WO-A-2004/026879
- WO-A-2004/108665
- WO-A-2005/012300
- WO-A-2005/016931
- WO-A-2005/100364
- WO-A-2006/042481
- WO-A-2006/137628
- WO-A-2007/108604
- US-A- 4 847 265
- US-A1- 2004 024 011
- US-A1- 2005 059 696
- US-A1- 2006 205 766
- US-B1- 6 429 210

## Description

### Technical field

The present invention belongs to the field of organic chemistry and relates to a new process for the synthesis of clopidogrel hydrogen sulfate of polymorphic form I.

Clopidogrel - preferably its pharmaceutically acceptable salts - is used for inhibiting platelet aggregation or decreasing the frequence of atherosclerotic events (brain strokes or cardiac attacks, mortality due to blood vessel disease) in patients with a symptomatic form of atherosclerotic disease such as recovered brain stroke, myocardial infarct and peripheral arterial occlusive disease.

### Technical problem

There exists a need for a new economical process for the synthesis of clopidogrel and of new stable forms of pharmaceutically acceptable salts thereof, which may be used in the preparation of pharmaceutical formulations. There exists also a need for an active ingredient having appropriate properties, such as flowability, particle size, particle distribution, since these properties affect the processability, content, stability and quality of the pharmaceutical formulations.

### Prior art

Clopidogrel, having the chemical name (S)-(+)-(2-chlorophenyl)-6,7-dihydro-4H-thieno[3,2-c]pyridin-5-yl acetic acid methyl ester, is described in US 4,847,265.

### Description of the invention

An object of the invention is a process designated as Process A for the preparation of a polymorphic form I of clopidogrel hydrogen sulfate with suitable properties in view of preparing a pharmaceutical formulation. We have surprisingly found that the temperature and duration of addition of the sulfuric acid solution in an organic solvent, which is added to the clopidogrel solution in the same organic solvent substantially affects the particle size of the obtained clopidogrel sulfate. For the preparation of a suitably stable and technologically controllable pharmaceutical formulation in view of the selected technology, particles of suitable morphological properties and flowability are required. The morphological properties can be characterized by the particle size distribution, which can be e.g. bimodal or unimodal, or by the mean particle size of the whole population measured by laser diffraction. By the process of the present invention the particles with the desired distribution can be obtained by controlling the crystallization conditions.

The Process A according to the invention for the preparation of the polymorphic form I of clopidogrel hydrogen sulfate comprises:
i) adding a sulfuric acid solution in an organic solvent at T2 to a clopidogrel solution in an organic solvent at T1 to obtain a suspension,
ii) stirring the suspension after completed addition for a certain time at T3,
iii) heating the suspension to T4 under control with a certain heating speed and stirring at this temperature for a certain time,
iv) isolating the precipitated product,
wherein the clopidogrel hydrogen sulfate of polymorphic form I is characterized by a mean particle size larger than 20 µm, T1 to T3 are temperatures which can be the same or different and are selected from -20 to 40°C, and T4 is a temperature from 15 to 40°C.

According to process A with the selection of particular technological parameters of crystallization according to the present invention the particles with appropriate flowable and morphological characteristics are obtained. Key technological parameters for the preparation of stable and easily technologically controlled substance suitable for the preparation of pharmaceutical composition are preferably the following, which can be independently selected from each other, and they form preferred embodiments of the process:
a) concentration of clopidogrel in organic solvent, which is between 0.1 mol/L do 1 mol/L,
b) concentration of sulfuric acid, which is between 1 mol/L to 4 mol/L, or concentrated sulfuric acid,
c) temperature of solution of added sulphuric acid - T2,
d) temperature of clopidogrel solution in organic solvent - T1,
e) temperature profile of crystallization step defined by temperatures T3 to T4,
f) duration time of adding solution of sulfuric acid in step i) and time of stirring of suspension in step ii), which is less than 1 hour,
g) moisture content in steps from i) to iv) during crystallization, which is between 0.1 w% to 1 w%.

Clopidogrel base used in the phase i) of the Process A is preferably extracted by a general known process into the same solvent as used in the steps i) through iv). The advantage is that no complete evaporation of the solvent is required during the extraction step which has a great influence on production time and purity of the final product. At the same time the redundant water is removed by partial destilation to the moisture content as defined in paragraph g).

The selected technological parameters as defined above increase the robustness of the process with the goal to prepare bigger particles of the final product.

The said selected technological parameters enable the preparation of particles with extraordinary flowable characteristics, wherein the tendency of clopidogrel salts toward electrostatic loading due to triboelectric effects is decreased, with at the same time the decrease of bulk volume below 6 mL/g.

The temperatures T1 to T3 can be the same or different and are selected within temperatures from -20 to 40°C, preferably from - 10 °C to 10 °C. The temperature T4 can be from 15 °C to 40 °C, preferably from 15 °C to 25 °C, and more preferably from 20°C to 25°C.

The organic solvents can be aliphatic C₃-C₁₀ alcohols, C₃-C₈ esters, C₃-C₁₅ ketones or C₄ to C₁₅ ketone, preferably higher ketones, most preferably methyl isobutyl ketone.

The molar ratio between the added acid and the clopidogrel base is preferably between 0.8 and 1.2, in particular between 0.95 and 1.05.

We have found that the temperature at which a sulfuric acid solution in an organic solvent reacts with a clopidogrel solution in the same organic solvent decisively defines the kinetics of nucleation and agglomeration of the precipitate, which in connection with the manner and turbulence of stirring directly defines the particle size of clopidogrel hydrogen sulfate. For example, the addition of a cold sulfuric acid solution in an organic solvent into a cold clopidogrel solution in an organic solvent results in larger particles and agglomerates, whereas the addition of a sulfuric acid solution that was heated to room temperature prior to the addition into the clopidogrel solution in an organic solvent generally results in smaller particles, though said example is not limiting. On the basis of experience and a precise knowledge of system properties, in a controlled manner there can be prepared particles of clopidogrel hydrogen sulfate of form I with a mean size larger than 20 µm, preferably larger than 30µm, more preferably larger than 50 µm and most preferably larger than 70 µm.

Thus, the above process is capable of producing such particles which form an embodiment of the invention.

Also disclosed is the preparation of polymorphic form I of clopidogrel hydrogen sulfate wherein it is prepared by the Process A and the product is washed during the isolation phase by an organic solvents containing dissolved sulfuric acid, wherein the organic solvents can be branched, straight-chain and/or cyclic hydrocarbons, preferably C₃-C₈ esters, C₄-C₁₅ or C₃ to C₁₅ ketones, preferably higher ketones, most preferably ethyl acetate and methyl isobutyl ketone. The concentration of sulfuric acid can be 0-4 mg/mL, preferably 0-2 mg/mL.

Thus, crystalline polymorphic form I clopidogrel hydrogen sulfate having a bulk volume of less than 3 mL/g is also disclosed.

Also disclosed are pharmaceutical formulations containing the above described clopidogrel hydrogen sulfate prepared according to the invention having a mean particle size of greater than 20µm. As pharmaceutically acceptable auxiliaries, substances generally known to one skilled in the art of pharmacy can be used.

The solid pharmaceutical compositions preferably have a dissolution profile of not less then 70 %(Q) in 0.1 M Hydrochloric acid in 30 minutes.

The formulations of the clopidogrel salts can be prepared by the use of known technological processes such as direct tabletting, wet granulation (with organic solvents e.g. MeOH, EtOH, acetone, isopropyl alcohol or mixtures thereof), thermoplastic granulation, dry granulation or lyophilization.

### Description of the Drawings

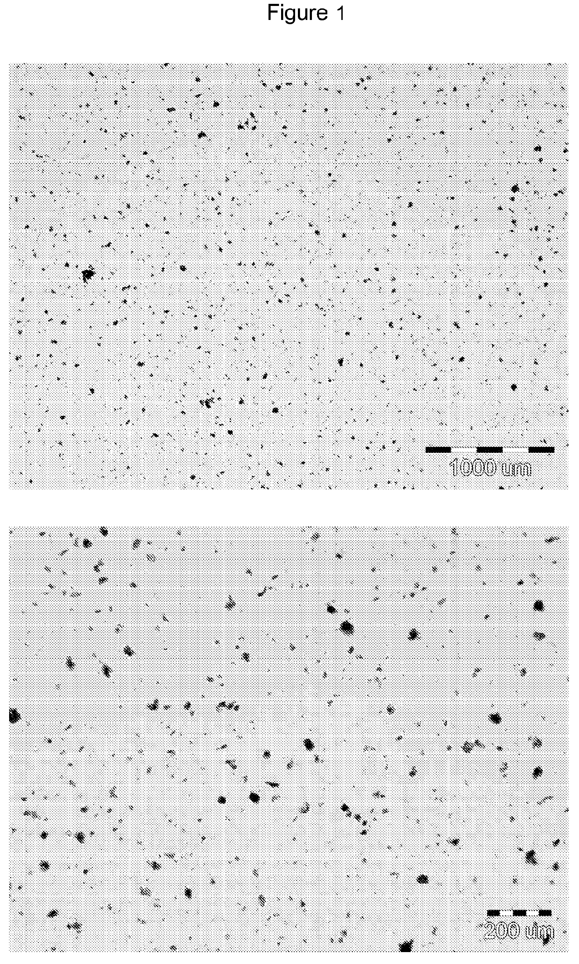

Fig. 1: microscopic image of particles obtained according to Example 1A,

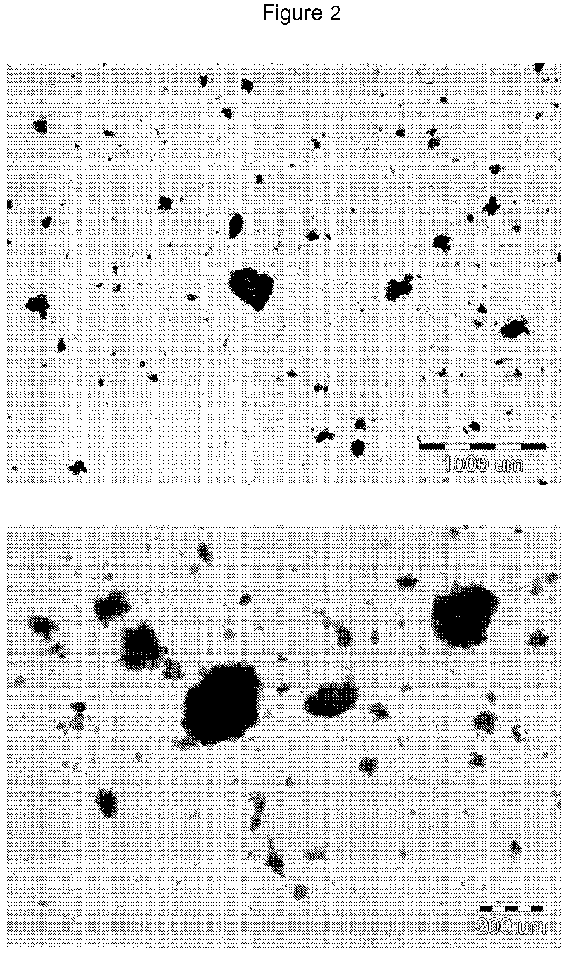

Fig. 2: microscopic images of particles obtained according to Example 1B.

X-ray powder diffractograms were recorded on a Philips PW3040/60 X'Pert PRO diffractometer by the use of CuK_{α}-radiation 1.541874, and FT-IR spectra on a Perkin-Elmer Spectrum 1000 apparatus with the KBr method.

Microscopic images were recorded with a Olympus BX 50 microscope, equipped with a Olympus DP70 camera. The mean particle sizes were measured on a Malvern, Mastersizer 2000 device in oil as the dispersion medium. The bulk volume measurements were performed according to European Pharmacopeia, chapter 2.9.15., Bulk volumes.

The present invention is illustrated by the following nonlimiting Examples.

### Examples

### Clopidogrel hydrogen sulfate

### Example 1A

7.05 g of clopidogrel base were dissolved in 100 mL of methyl isobutyl ketone (MIBK), 1.1 mL of dichloromethan was added thereto and the solution was cooled to -7 °C. A sulfuric acid solution was prepared at -10 °C by adding 2.5 mL of 98 % sulfuric acid to 100 mL of MIBK in about 30 minutes. 40 mL of the prepared sulfuric acid solution were added to the solution of clopidogrel base at -7 °C in 2 hours. At the time of addition to the clopidogrel base solution, the sulfuric acid solution was heated from a temperature between -5 °C and 5 °C to the temperature of 20 °C. The suspension was stirred at -7 °C for 3 hours, then it was heated to 17 °C within 8 hours and then to 20 °C within further 10 hours. The product was filtered on a vacuum filter and washed with MIBK. The product was dried in a flow of dry air for some hours and then heated to 50 °C and dried in vacuum for 3 hours. 7.48 g of a dry product (82 % yield) were obtained. A microscopic image of the particles of the obtained product is shown in Fig. 10. The mean particle size is 35 µm with the following particle size distribution: 10 % of particles smaller than 12.2 µm, 50 % of particles larger than 31.7 µm and 90 % of particles smaller than 60.8µm.

### Example 1B

3 kg of clopidogrel hydrogen sulfate were suspended in 13 L of MIBK. 20 L of a saturated NaHCO₃ solution were added thereto and the clopidogrel base was extracted with MIBK. The extraction of water phase was repeated with 8 L of MIBK. The organic layers were combined, washed with water and evaporated to an oily residue. The oily residue was dissolved with 32 L of MIBK and 0.32 L of methylene chloride were added thereto, whereupon the solution was cooled to -7 °C. In another vessel 0.34 L of 98 % sulfuric acid was dissolved in 13 L of MIBK at -10 °C within 1 hour. The acid solution was maintained at the temperature of -7 °C and then it was added to the solution of clopidogrel base within 3 hours. At the time of addition the temperature of the sulfuric acid solution was maintained below 0 °C. After completed acid addition the suspension was stirred for 3 hours, then heated to 17 °C within 8 hours, whereupon the temperature was raised to 25°C in the next 10 hours. The suspension was filtered on a vacuum filter and washed twice with 10 L of MIBK. The product was dried under the flow of dry nitrogen for 3 hours and sieved. Then the product was dried at 50-60 °C for 5 hours and sieved again. 2.165 kg of the product were obtained. A microscopic image of the particles of the obtained product is shown in Fig. 11. The mean particle size was 75 µm with the following particle size distribution: 10 % of particles smaller than 8.6 µm, 50 % of particles larger than 52.3 µm and 90 % of particles smaller than 130.5 µm.

### Example 1C

1 g of clopidogrel hydrogen sulfate was dissolved in 1 mL of methanol and to the solution 1.5 mL of diethyl ketone were added. The solution was slowly evaporated to dryness under reduced pressure at a temperature of 50 °C. The obtained product was characterized by FT-IR spectrum: IR spectrum (cm⁻¹) : 590, 721, 761, 884, 1043, 1079, 1189, 1223, 1321, 1436, 1479, 1652, 1750, 2560 and 2924 cm⁻¹. Chromatographic HPLC purity was greater than 99.5 %.

### Example 1D

260 g of clopidogrel 2-propyl sulfate were suspended in 2100 mL of methyl isobutyl ketone and 780 mL of water were added. The suspension was stirred and pH was set to 7.5 to 8 with slow addition of 1M NaOH. The obtained mixture was stirred and phases were separated. The organic layer was thoroughly washed with 780 mL of water about 3 times. The solution of clopidogrel base in methyl isobutyl ketone was evaporated by vacuum distillation until 90 % of solvent were removed. The amount of clopidogrel base was determined and dissolved in methyl isobutyl ketone so that the total amount of solvent was 2600 mL. The solution was filtered and cooled to -7°C and 31 mL of methylene chloride were added. While stirring (overhead stirrer) the solution of 98% sulfuric acid (53 g) in 860 mL of methyl isobutyl ketone was added within about 3 hours. After this addition was complete, the suspension was stirred for about 3 hours at 0°C. Then the temperature was raised to 17 °C in 8 hours, followed by slow heating to 25 °C in 10 hours. Clopidogrel hydrogen sulfate form I was collected.
The material was dried in a fluid bed dryer at 20 and 30 °C. When the temperature of the material was constant, the product was sieved and the material was further dried while stepwise rising the air temperature to 50 - 55 °C. Yield: 89%, Characteristic XRPD peaks show polymorphic form I, HPLC purity 99.8, average particle size: 34 µm, bulk density: 2.5 mL/g, residual MIBK less then 2500 ppm.

### Example 1E

5.0 g (loss on drying 4.6%) of clopidogrel base were dissolved in 100 mL methyl isobutyl ketone and filtered trough 0.45 µm filter into a 250 mL reactor equipped with an overhead stirrer. The clear solution was cooled to -7 °C. While stirring at that temperature 0.76 mL of 98% sulphuric acid were drop-wise added to the solution in 3 hours. A suspension was formed and stirring at -7°C was continued for 3 hours after the addition. Then the temperature was slowly raised to 25 °C in 8 hours, and nucleation was observed. The suspension was stirred for 10 hours at 25°C to get well crystallized clopidogrel hydrogen sulfate form I suspension. The product was collected, washed with 15 mL of acetone, dried in a vacuum dryer at 25 °C for 10 hours and sieved trough a rough grid. Then the drying was continued and the temperature was raised to 30 °C for 2 hours, followed by drying at temperature of 40°C for 3 hours, and subsequently the product was sieved through a fine grid. Finally the product was dried for 4 hours at 50°C. Yield: 5.2 g, XRPD: form I, average particle size: 50 µm. Bulk density: 3.0 mL/g, HPLC purity: 99.7%, residual MIBK 400 ppm.

## Claims

1. A process for the preparation of clopidogrel hydrogen sulfate of polymorphic form I which is **characterized by** a mean particle size larger than 20 µm comprising:
i) adding a sulfuric acid solution in an organic solvent at T2 to a solution of clopidogrel in an organic solvent at T1,
ii) stirring the suspension after completed addition at T3 for a certain time,
iii) heating the suspension to T4 under control with a certain heating speed and stirring at this temperature for a certain time,
iv) isolating the precipitated product,
wherein T1 to T3 are temperatures which can be the same or different and are selected from -20 to 40°C, and T4 is a temperature from 15 to 40°C.

2. Process according to claim 1, wherein the clopidogrel hydrogen sulphate of polymorphic form I is **characterized by** a mean particle size larger than 50 µm.

3. Process according to claim 2, wherein the clopidogrel hydrogen sulphate of polymorphic form I is **characterized by** a mean particle size larger than 70 µm.

4. Process according to any one of claims 1 to 3, wherein the organic solvent is a C₄ to C₁₅ ketone.

5. Process according to claim 4, wherein the organic solvent is methyl isobutyl ketone.

6. Process according to any one of claims 1 to 5, wherein the solution of clopidogrel contains methyl isobutyl ketone and methylene chloride.

7. Process according to any one of claims 1 to 6, wherein the temperatures T1 to T3 can be the same or different and are selected within temperatures from -10°C to 10°C.

8. Process according to any one of claims 1 to 7, wherein the temperature T4 is from 15°C to 25°C.

9. Process according to claim 8, wherein the temperature T4 is from 20°C to 25°C.

10. Process according to any one of claims 1 to 9, wherein the concentration of clopidogrel in organic solvent is between 0.1 mol/l to 1 mol/l.

11. Process according to any one of claims 1 to 10, wherein the duration time of adding solution of sulfuric acid in step i) and time of stirring of suspension in step ii) is less than 1 hour.

12. Process according to any one of claims 1 to 11, wherein the molar ratio between the added acid and the clopidogrel is between 0.8 and 1.2.

13. Process according to claim 12, wherein the molar ratio between the added acid and the clopidogrel is between 0.95 and 1.05.

## Patentansprüche

1. Verfahren zur Herstellung von Clopidogrelhydrogensulfat polymorpher Form I, welches durch eine mittlere Teilchengröße von größer als 20 µm gekennzeichnet ist, bei dem:
i) eine Schwefelsäurelösung in einem organischen Lösungsmittel bei T2 zu einer Lösung von Clopidogrel in einem organischen Lösungsmittel bei T1 zugegeben wird,
ii) nach vollständiger Zugabe die Suspension bei T3 für eine gewisse Zeit gerührt wird,
iii) die Suspension mit einer gewissen Aufheizgeschwindigkeit gesteuert auf T4 erwärmt wird und bei dieser Temperatur für eine gewisse Zeit gerührt wird,
iv) das ausgefallene Produkt isoliert wird,
wobei T1 bis T3 Temperaturen sind, die gleich oder unterschiedlich sein können und von -20 bis 40°C ausgewählt sind, und T4 eine Temperatur von 15 bis 40°C ist.

2. Verfahren nach Anspruch 1, bei dem das Clopidogrelhydrogensulfat polymorpher Form I durch eine mittlere Teilchengröße von größer als 50 µm gekennzeichnet ist.

3. Verfahren nach Anspruch 2, bei dem das Clopidogrelhydrogensulfat polymorpher Form I durch eine mittlere Teilchengröße von größer als 70 µm gekennzeichnet ist.

4. Verfahren nach einem der Ansprüche 1 bis 3, bei dem das organische Lösungsmittel ein C₄- bis C₁₅-Keton ist.

5. Verfahren nach Anspruch 4, bei dem das organische Lösungsmittel Methylisobutylketon ist.

6. Verfahren nach einem der Ansprüche 1 bis 5, bei dem die Lösung von Clopidogrel Methylisobutylketon und Methylenchlorid enthält.

7. Verfahren nach einem der Ansprüche 1 bis 6, bei dem die Temperaturen T1 bis T3 gleich oder unterschiedlich sein können und aus Temperaturen von -10°C bis 10°C ausgewählt sind.

8. Verfahren nach einem der Ansprüche 1 bis 7, bei dem die Temperatur T4 von 15°C bis 25°C ist.

9. Verfahren nach Anspruch 8, bei dem die Temperatur T4 von 20°C bis 25°C ist.

10. Verfahren nach einem der Ansprüche 1 bis 9, bei dem die Konzentration von Clopidogrel in organischem Lösungsmittel zwischen 0,1 Mol/l bis 1 Mol/l beträgt.

11. Verfahren nach einem der Ansprüche 1 bis 10, bei dem die Dauer der Zugabe von Schwefelsäurelösung in Schritt i) und die Zeit des Rührens der Suspension in ii) weniger als 1 Stunde beträgt.

12. Verfahren nach einem der Ansprüche 1 bis 11, bei dem das Molverhältnis zwischen der zugesetzten Säure und dem Clopidogrel zwischen 0,8 und 1,2 beträgt.

13. Verfahren nach Anspruch 12, bei dem das Molverhältnis zwischen der zugesetzten Säure und dem Clopidogrel zwischen 0,9 und 1,05 beträgt.

## Revendications

1. Procédé de préparation d'un hydrogénosulfate de clopidogrel de forme polymorphe I, qui est **caractérisé par** une taille moyenne de particules supérieure à 20 µm, comprenant les étapes suivantes :
i) ajouter une solution d'acide sulfurique dans un solvant organique, à T2, à une solution de clopidogrel dans un solvant organique, à T1,
ii) une fois cette addition achevée, agiter la suspension à T3 pendant un certain temps,
iii) chauffer la suspension à T4, en réglant ce chauffage à une certaine vitesse, et l'agiter à cette température pendant un certain temps,
iv) et isoler le produit précipité,
étant entendu que les T1 à T3 sont des températures qui peuvent être identiques ou différentes et qui sont choisies dans l'intervalle allant de -20 °C à 40 °C, et que T4 est une température qui vaut de 15 °C à 40 °C.

2. Procédé conforme à la revendication 1, dans lequel l'hydrogénosulfate de clopidogrel de forme polymorphe 1 est **caractérisé par** une taille moyenne de particules supérieure à 50 µm.

3. Procédé conforme à la revendication 2, dans lequel l'hydrogénosulfate de clopidogrel de forme polymorphe 1 est **caractérisé par** une taille moyenne de particules supérieure à 70 µm.

4. Procédé conforme à l'une des revendications 1 à 3, dans lequel le solvant organique est une cétone en C₄ à C₁₅.

5. Procédé conforme à la revendication 4, dans lequel le solvant organique est de la méthyl-isobutyl-cétone.

6. Procédé conforme à l'une des revendications 1 à 5, dans lequel la solution de clopidogrel contient de la méthyl-isobutyl-cétone et du chlorure de méthylène.

7. Procédé conforme à l'une des revendications 1 à 6, dans lequel les températures T1 à T3 peuvent être identiques ou différentes et sont choisies dans l'intervalle de températures allant de -10 °C à 10 °C.

8. Procédé conforme à l'une des revendications 1 à 7, dans lequel la température T4 vaut de 15 °C à 25 °C.

9. Procédé conforme à la revendication 8, dans lequel la température T4 vaut de 20 °C à 25 °C.

10. Procédé conforme à l'une des revendications 1 à 9, dans lequel la concentration de clopidogrel dans le solvant organique vaut entre 0,1 mol/L et 1 mol/L.

11. Procédé conforme à l'une des revendications 1 à 10, dans lequel la durée de l'addition de la solution d'acide sulfurique dans l'étape (i) et le temps d'agitation de la suspension dans l'étape (ii) valent moins de 1 heure.

12. Procédé conforme à l'une des revendications 1 à 11, dans lequel le rapport molaire entre l'acide ajouté et le clopidogrel vaut entre 0,8 et 1,2.

13. Procédé conforme à la revendication 12, dans lequel le rapport molaire entre l'acide ajouté et le clopidogrel vaut entre 0,95 et 1,05.
